# EUROPEAN PATENT SPECIFICATION

(11) **EP 0 813 885 B1**
(45) Date of publication and mention of the grant of the patent: **25.05.2005**
(21) Application number: 97109537.7
(22) Date of filing: 12.06.1997
(51) Int. Cl.: A61N 1/05

(54) **An electrode for biomedical use, for example, for cardiac stimulation**
Elektrode für biomedizinische Zwecke, zum Beispiel für Herzstimulation
Electrode à usage biomédical, pour stimuler le coeur par exemple

(30) Priority: 17.06.1996 IT TO960519
(43) Date of publication of application: 29.12.1997
(73) Proprietor: SORIN BIOMEDICA CRM S.r.l., 13040 Saluggia (Vercelli) (IT)
(72) Inventor: Arru, Pietro, 10020 Marentino (Torino) (IT); Garberoglio, Bruno, 10156 Torino (IT); Rolando, Giovanni, 10043 Chivasso (Torino) (IT); Santi, Marco, 10036 Settimo Torinese (Torino) (IT)
(74) Representative: Bosotti, Luciano

(56) References cited:
- EP-A- 0 224 080
- EP-A- 0 291 476
- US-A- 4 542 752
- US-A- 4 612 100
- US-A- 4 934 381
- GUPTA B K ET AL: "MICROMECHANICAL PROPERTIES OF AMORPHOUS CARBON COATINGS DEPOSITED BY DIFFERENT DEPOSITION TECHNIQUES" THIN SOLID FILMS, vol. 270, no. 1/02, 1 December 1995 (1995-12-01), pages 391-398, XP000595248 ISSN: 0040-6090

## Description

The present invention relates to electrodes for biomedical use and has been developed with particular attention to its possible use in connection with cardiac stimulation electrodes. However, the present invention is suitable for use for the production of electrodes of any type for biomedical use, for example, for electrocardiographic or electroencephalographic use, etc.

In this field of use, and particularly with regard to cardiac stimulation, the use of metal electrodes based on, for example, porous platinum or smooth platinum-iridium has for some time been confirmed as the preferred selection.

The use of electrodes of this type achieves excellent results in terms of stimulation impedance (which is typically of the order of about 500 ohms for electrode surfaces of the order of 6-8 mm²) and also with regard to the stimulation-threshold characteristics.

In this connection, it is well known that, in the days immediately following implantation in the wearer's body, the stimulation threshold (in practice the level of voltage which has to be applied to the electrode to achieve effective stimulation) increases until it reaches a peak value and then falls gradually in the following days to an asymptotic value (the so-called "chronic threshold") which is maintained during the normal use of the electrode. The stimulation-threshold value, particularly the chronic threshold value, is clearly related to the energy absorption during the performance of the stimulation function; a lower threshold value corresponds to a lower energy absorption and hence to a longer life of the supply sources (batteries) of the stimulation device.

To prevent these phenomena from arising and/or in any case to reduce their incidence, so-called steroid eluting electrodes have recently been introduced. In this solution, the electrode, as implanted, brings with it a certain quantity of steroid agents (typically corticosteroids) which can oppose local inflammatory phenomena considered to be amongst the main factors responsible for the initial rise in the stimulation threshold. It has also been found that these electrodes have a lower chronic threshold.

The object of the present invention is to provide an electrode for biomedical use and particularly, but not exclusively, for cardiac stimulation, which combines excellent characteristics, for example, in terms of stimulation impedance and implantation compatibility, with a uniform stimulation-threshold curve, which does not have the initial peak characteristic of conventional metal electrodes and which also shows a lower chronic threshold value.

According to the present invention, this object is achieved by virtue of an electrode having the specific characteristics recited in the following claims.

Essentially, the present invention is based upon the solution of coating the electrode for biomedical use with a layer of carbon material which has a turbostratic structure, preferably applied by cathode sputtering.

Carbon coatings of this type are used widely in the biomedical field as shown by the documents EP-A-0 102 328, EP-A-0 224 080 and EP-A-0 291 476, amongst others.

In all of these prior documents, the use of a carbon coating is connected essentially with the intention of conferring good biocompatibility (haemocompatibility) characteristics on the substrate (which is usually constituted by an implant such as a heart-valve prosthesis, a vascular prosthesis and/or components of these prostheses).

Electrodes for biomedical use which have a coating of carbon material on at least a portion of their surface are known from US-A-4 612 100, US-A-4 934 381 or US-A-4 542 752.

The present invention is based upon the recognition of the wholly unexpected fact that, if a coating having a turbostratic structure is applied to an electrode, particularly a metal electrode such as a porous Pt or a smooth Pt-It electrode, it affects its performance, particularly with regard to the reduction and the elimination of the undesired initial increase in the stimulation threshold.

Although the physical/chemical mechanism upon which this behaviour is based is not entirely clear, the Applicant has found that it is reproduced in wholly deterministic terms and is not linked to any uncertain and/or imponderable parameter. Moreover, with regard to the electrical characteristics such as the stimulation impedance, the use of a coating of carbon material enables stimulation impedance values of the order of 1000-1200 ohms to be achieved with electrode surface values of the order of 2-2.5 mm². Naturally this is combined with the well-known biocompatible nature of carbon coating materials so that the coating can also be extended beyond the actual electrode to the adjacent parts and virtually to the entire catheter of which the electrode forms part. This also applies in particular to the parts made of non-metallic materials such as silicone and other plastics materials, with the further advantage of conferring a certain degree of lubrication on the surfaces of these parts, which facilitates their insertion by catheterization.

With specific regard to the electrode, explicit reference will be made in the following description to a layer of carbon material applied by sputtering techniques such as those described in the three prior documents cited in the introductory part of the description. It should, however, be noted that - as is well known to experts in the art - generally similar carbon coatings can also be applied by different techniques such as, for example, high-temperature pyrolysis of a hydrocarbon, which technique is currently used for the production of pyrolytic carbon (also known commercially as LTI carbon) or low-temperature coating techniques such as the catalytic decomposition of a gaseous carbon precursor (used for the decomposition of materials known commercially as "ULTI carbons") or similar techniques about which there is extensive literature also in the patent field.

The invention will now be described, purely by way of non-limiting example, with reference to the appended drawings, in which:
Figure 1 shows schematically an electrode for biomedical use formed according to the present invention, and
Figure 2 is a graph showing some operating characteristics of an electrode according to the invention.

In the drawings, an electrode for biomedical use, generally indicated 1, is shown - purely by way of example - in the form of a cardiac stimulation electrode.

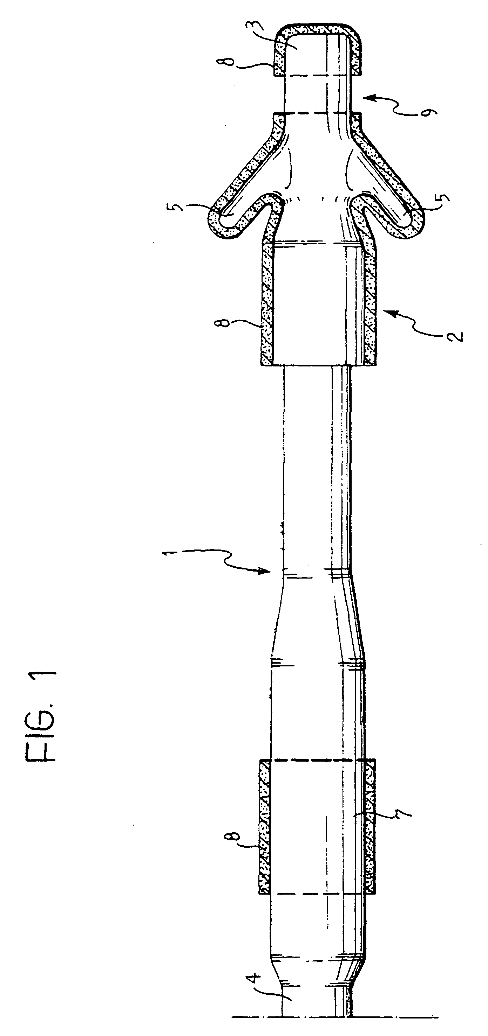

As can readily be appreciated by a person skilled in the art, the drawings, and, in particular, Figure 1, show essentially the distal end of the electrode, the rest of the electrode (and in particular, its proximal portion) being of a structure well known in the art which does not need to be described in detail herein and, moreover, is not relevant for the purposes of an understanding of the invention.

Within the electrode 1 it is possible, in general, to distinguish a distal end 2 on the tip of which there is a stimulation tip 3 (or actual electrode) made of an electrically-conductive material, for example, a metal such as porous Pt or smooth Pt-Ir.

For clarity, it should be pointed out that, although the term "electrode" is correctly applied to the tip indicated 3, in normal usage, the term is also used to indicate the assembly indicated by the reference numeral 1 and the further elements normally associated therewith, that is, the device currently known as an electrocatheter.

In accordance with widely-known criteria, the end 2 on which the electrode 3 is mounted is constituted by a tubular body with radial dimensions slightly larger than the shaft 4 of the catheter which is used to insert the electrode 3 in the stimulation position.

The wire or wires which carry the electrical-stimulation energy to the electrode 3 extend within the shaft 4 and the end 2. One or more appendages 5, usually formed integrally with the end 2, extend like the arms of an anchor from the body of the end 2, which is usually made of a biocompatible plastics material (for example, silicone rubber), and are intended to cooperate in the manner of an anchor with the tissue of the region of the body in which the electrode 3 is implanted in order to hold the electrode 3 in place.

A further electrode having a generally collar-like shape and indicated 7 is applied to the shaft 4 a certain distance from the end 2 for cooperating with the electrode 3 in the stimulation operation, particularly for detecting the propagation of the stimulation pulse in the heart muscle.

In this connection, it should again be mentioned that although the present description refers specifically to a cardiac stimulation electrode, the solution according to the invention is not limited absolutely to this use or to the geometry just described or, in particular, to the simultaneous presence of two or more electrodes such as the electrodes 3 and 7 described above.

The structure which has just been described with reference to Figure 1 is known in the art and does not require further description of specific constructional details which, moreover, are not relevant or important for the purposes of an understanding of the implementation of the invention.

The main characteristic of the invention is that a coating 8 of turbostratic carbon material is applied to at least a portion of the electrode 3 (and/or of the electrode 7), the coating preferably extending over the entire exposed surfaces of the electrodes 3 and 7 and also over all or part of the surfaces both of the enlarged end 2 and of the shaft 4 of the catheter for the insertion of the electrode, particularly over the appendages 5.

Since, as is known and as will be described further below, this coating has characteristics of electrical conductivity, the coating 8 is generally interrupted adjacent the electrode 3 and/or the electrode 7, for example, by annular portions 9 of untreated silicone rubber, in order to insulate the electrode 3 and/or the electrode 7 from adjacent coated portions.

The coating 8 is preferably constituted by a coating of carbon material formed according to the teachings given in any one of the European patent applications cited in the introductory part of the present description.

Naturally - as already stated - the coating 8 may also be applied by other known techniques. The solution described in applications EP-A-0 102 328, EP-A-0 224,080 and EP-A-0 291 476, however, has been found particularly advantageous from various points of view, particularly with regard to the fact that the coating 8 can be applied both to metallic materials, and hence to materials with high melting points (for example, porous Pt or Pt-Ir), and to plastics materials with low softening points such as the silicone rubber which coats the end 2 and/or the shaft 4.

Moreover, the solution described in the European patent applications cited has been found advantageous as regards the intimate adherence of the coating to whatever substrate is used, particularly when it has characteristics of flexibility. Moreover, this solution has the advantage of enabling the deposition parameters and the final characteristics of the carbon coating 8 to be regulated precisely, particularly with regard to the production of a coating having the following characteristics:
- a turbostratic structure,
- crystal dimensions less than about 100 Angstroms,
- thickness of between about 0.3 and about 1.0 microns,
- electrical resistivity of less than about 1 ohm*cm.
- adhesion to the substrate greater than 70 MPa (measured by the "pull test") and greater than 6N/cm (measured by the "tape test").

Tests carried out by the Applicant show that the parameters given above should be considered, on the one hand, broadly preferable but, on the other hand, not absolutely essential.

This applies in particular with regard to the parameters such as the thickness of the coating and the dimensions of the crystallites.

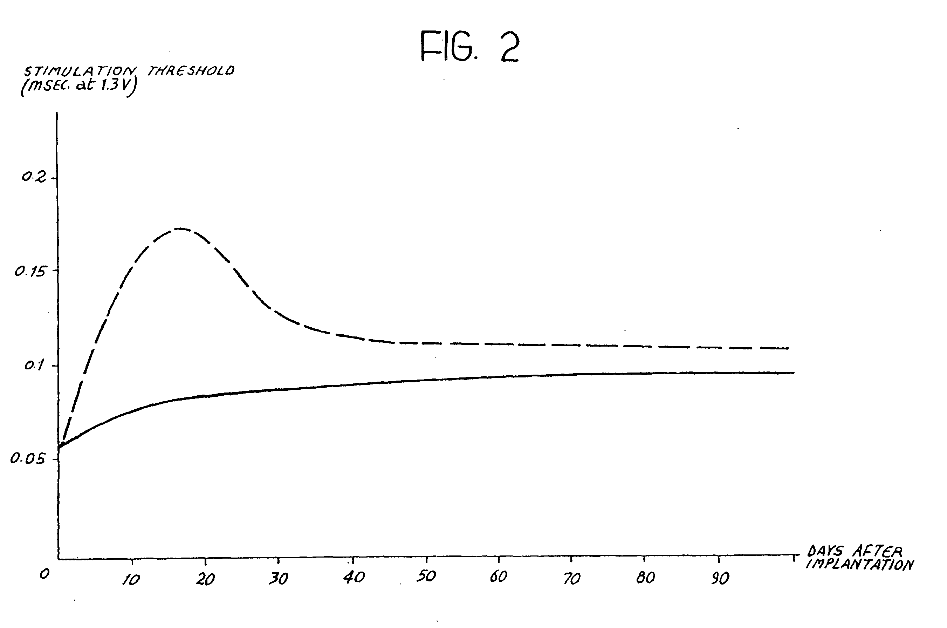

The graph of Figure 2 shows a typical curve of the stimulation threshold value (volts, on the ordinate) as a function of time (days, on the abscissa) detected - in sheep - with an electrode according to the invention (continuous line), compared with the curve detected in identical conditions - for a conventional metal electrode (porous platinum) (broken line).

Naturally, the principle of the invention remaining the same, the details of construction and forms of embodiment may be varied widely with respect to those described and illustrated, without thereby departing from the scope of the present invention as defined in the claims.

## Claims

1. An electrode (3, 7) for biomedical use which has a coating (8) of carbon material on at least a portion of its surface, **characterized in that** the carbon material has a turbostratic structure.

2. An electrode according to Claim 1, **characterized in that** the carbon material has crystallites with dimensions of less than 100 Angstroms.

3. An electrode according to any one of Claims 1 to 2, **characterized in that** the coating (8) has a thickness of between about 0.3 and 1 microns.

4. An electrode according to any one of the preceding claims, **characterized in that** the carbon material has an electrical resistivity of less than about 1 ohm*cm.

5. An electrode according to any one of the preceding claims, **characterized in that** the adhesion of the coating to the said portion is greater than 70 MPa and greater than 6N/cm, measured by the "pull test" and by the "tape test", respectively.

6. An electrode according to any one of the preceding claims, **characterized in that** the coating (8) is applied by cathode sputtering.

7. An electrode according to any one of the preceding claims, **characterized in that** the said portion of the surface is made of metallic material.

8. An electrode according to Claim 7, **characterized in that** the metallic material is selected from the group constituted by porous Pt and smooth Pt-Ir.

9. An electrode according to any one of the preceding claims, **characterized in that** it has associated support means (4) of electrically non-conductive material, and **in that** the coating of carbon material (8) is also applied to at least a portion of the support means (4).

10. An electrode according to Claim 9, **characterized in that** the support means (4) jointly define a catheter for inserting the electrode (3) in position.

11. An electrode according to Claim 10, **characterized in that** a further electrode (7) is mounted on the insertion catheter, and **in that** at least part of the further electrode is also provided with a surface coating of the carbon material (8).

## Patentansprüche

1. Elektrode (3, 7) zur biomedizinischen Verwendung, welche eine Beschichtung (8) aus Kohlenstoffmaterial auf wenigstens einem Teil ihrer Oberfläche aufweist, **dadurch gekennzeichnet, dass** das Kohlenstoffmaterial eine turbostatische Struktur aufweist.

2. Elektrode nach Anspruch 1, **dadurch gekennzeichnet, dass** das Kohlenstoffmaterial Kristallite mit Abmessungen von weniger als 100 Angstrom aufweist.

3. Elektrode nach irgendeinem der Ansprüche 1 bis 2, **dadurch gekennzeichnet, dass** die Beschichtung (8) eine Dicke von ungefähr zwischen 0,3 und ein Mikron aufweist.

4. Elektrode nach irgendeinem der vorangehenden Ansprüche, **dadurch gekennzeichnet, dass** das Kohlenstoffmaterial einen elektrischen Widerstand von wenigster als ungefähr 1 Ohm*cm aufweist.

5. Elektrode nach irgendeinem der vorangehenden Ansprüche, **dadurch gekennzeichnet, dass** die Anhaftung der Beschichtung zu dem Teil größer ist als 70 Mpa und größer als 6 N/cm, jeweils gemessen durch den "Zugtest" und durch den "Bandtest".

6. Elektrode nach irgendeinem der vorangehenden Ansprüche, **dadurch gekennzeichnet, dass** die Beschichtung (8) durch Kathodenzerstäubung angebracht wird.

7. Elektrode nach irgendeinem der vorangehenden Ansprüche, **dadurch gekennzeichnet, dass** der Teil der Oberfläche aus metallischem Material hergestellt ist.

8. Elektrode nach Anspruch 7, **dadurch gekennzeichnet, dass** das metallische Material aus der Gruppe ausgewählt wird, die gebildet wird durch poröses Pt und glattes Pt-Ir.

9. Elektrode nach irgendeinem der vorangehenden Ansprüche, **dadurch gekennzeichnet, dass** sie zugeordnete Stützmittel (4) aus elektrisch nicht leitfähigem Material aufweist und dadurch, dass die Beschichtung aus Kohlenstoffmaterial (8) ebenso auf wenigstens einem Teil der Stützmittel (4) angebracht ist.

10. Elektrode nach Anspruch 9, **dadurch gekennzeichnet, dass** die Stützmittel (4) gemeinsam einen Katheter vorgeben zur Einbringung der Elektrode (3) in Position.

11. Elektrode nach Anspruch 10, **dadurch gekennzeichnet, dass** eine weitere Elektrode (7) auf dem Einfügungskatheter angebracht ist und dadurch, dass wenigstens ein Teil der weiteren Elektrode ebenso mit einer Oberflächenbeschichtung des Kohlenstoffmaterials (8) ausgestattet ist.

## Revendications

1. Une électrode (3, 7) à usage biomédical, qui possède un revêtement (8) de matériau en carbone sur au moins une partie de sa surface, **caractérisée en ce que** le matériau en carbone a une structure turbostratique.

2. Une électrode selon la revendication 1, **caractérisée en ce que** le matériau en carbone possède des cristallites ayant des dimensions inférieures à 100 angstroems.

3. Une électrode selon l'une des revendications 1 à 2, **caractérisée en ce que** le revêtement (8) présente un épaisseur comprise entre environ 0,3 et 1 micron.

4. Une électrode selon l'une des revendications précédentes, **caractérisée en ce que** le matériau en carbone présente une résistivité électrique inférieure à environ 1 ohm*cm.

5. Une électrode selon l'une des revendications précédentes, **caractérisée en ce que** l'adhérence du revêtement à ladite partie est supérieure à 70 MPa et supérieure à 6 N/cm, mesurée par "essai de traction" et "essai sur bande", respectivement.

6. Une électrode selon l'une des revendications précédentes, **caractérisée en ce que** le revêtement (8) est appliqué par pulvérisation cathodique.

7. Une électrode selon l'une des revendications précédentes, **caractérisée en ce que** ladite partie de la surface est réalisée en un matériau métallique.

8. Une électrode selon la revendication 7, **caractérisée en ce que** le matériau métallique est choisi dans le groupe constitué par le Pt poreux et le Pt-Ir lisse.

9. Une électrode selon l'une des revendications précédentes, **caractérisée en ce qu'**elle possède des moyens supports associés (4) en matériau électriquement non conducteur, et **en ce que** le revêtement de matériau en carbone (8) est également appliqué sur au moins une partie des moyens supports (4).

10. Une électrode selon la revendication 9, **caractérisée en ce que** les moyens supports (4) définissent ensemble un cathéter pour insertion en place de l'électrode (3).

11. Une électrode selon la revendication 10, **caractérisée en ce qu'**une autre électrode (7) est montée sur le cathéter d'insertion, et **en ce qu'**au moins un partie de l'autre électrode est également pourvue d'un revêtement de surface du matériau en carbone (8).
